# EUROPEAN PATENT APPLICATION

(11) **EP 3 789 023 A1**
(43) Date of publication of application: **10.03.2021**
(21) Application number: 19774894.0
(22) Date of filing: 05.03.2019
(51) Int. Cl.: A61K 31/341, A23L 33/10, A23K 20/121, A61P 25/28

(54) **COMPOSITION FOR PREVENTING OR TREATING COGNITIVE DISORDER-ASSOCIATED DISEASES, CONTAINING MUMEFURAL**

(30) Priority: 27.03.2018 KR 20180035324
(71) Applicant: Korea Institute of Oriental Medicine, Daejeon 34054 (KR)
(72) Inventor: JEON, Won Kyung, Seoul 01423 (KR); BANG, Jihye, Dalseong-gun Daegu 42968 (KR); KIM, Min Soo, Seoul 08596 (KR)
(74) Representative: Ström & Gulliksson AB
(86) International application number: PCT/KR2019/002545
(87) International publication number: WO 2019/190069

(57) **Abstract**

The present invention relates to a composition for preventing or treating a cognitive impairment-related disease, which includes mumefural, and more particularly, to a pharmaceutical composition; a health functional food composition; and a feed composition respectively for preventing or treating a cognitive impairment-related disease; a health functional food composition for enhancing learning ability, cognitive function, or memory, each composition including mumefural or an acceptable salt thereof as an active ingredient; and a method of treating a cognitive impairment-related disease using the pharmaceutical composition.

Due to excellent effects of the mumefural of the present invention on normalizing damage to the brain (*i.e*., basal forebrain, white matter, hippocampus, *etc*.) and excellent effects thereof on enhancing memory, the mumefural of the present invention may be effectively used to treat neurodegenerative diseases (*i.e*., dementia, Alzheimer's disease, *etc*.) and cognitive impairment caused by brain damage (*i.e*., memory impairment), and may be used to enhance learning ability, cognitive function, and memory.

## Description

### TECHNICAL FIELD

The present invention relates to a composition for preventing or treating a cognitive impairment-related disease, which includes mumefural, and more particularly, to a pharmaceutical composition; a health functional food composition; and a feed composition for preventing or treating a cognitive impairment-related disease, wherein each of the compositions includes mumefural or an acceptable salt thereof as an active ingredient; a health functional food composition for enhancing learning ability, cognitive function, or memory; and a method of treating a cognitive impairment-related disease using the pharmaceutical composition.

### BACKGROUND ART

Cognitive ability encompasses memory, spatiotemporal recognition ability, decision-making judgment, language ability, computation ability, *etc.*, and enables an individual to perform common activities of daily living without help from others. However, any brain damage such as sudden death of nerve cells caused by stroke or trauma or slow death of nerve cells causing degenerative brain diseases may directly result in irreversible functional disorders of a neural network. In the end, the cognitive abilities decrease causing forgetfulness or diseases such as memory impairment, dementia, and Alzheimer's disease, and common activities of daily living cannot be maintained. The number of patients with cognitive impairment-related diseases, which cause not only pain for individuals but also a decrease in working-age population, is expected to increase to 74.7 million by 2030 (Abate G, 2017, Oxid Med Cell Longev. 2017:10), and thus, there is still an urgent need for development of therapeutic agents or a treatment method therefor.

Various patent applications directed to compositions having effects on treatment of cognitive impairment and amelioration of cognitive ability have been published and registered to date in the Republic of Korea, and the published representative patent applications are as follows. Korean Laid-open Patent Application Publication No. 2014-0144785 discloses a composition for enhancing memory and learning ability, the composition including an extract from *Citrus Junos Tanaka* as an active ingredient; Korean Patent No. 10-1837444 discloses a composition for preventing, alleviating, or treating cognitive dysfunction, the composition including a *Potentilla fragarioides* extract as an active ingredient; Korean Patent No. 10-1823892 discloses *Impatiens balsamina* extracts for improvement of memory, improvement of cognitive ability, and prevention, delay, or treatment of dementia. Although such chemical drugs and natural compositions have been developed and are commercially available, agents having dramatic and obvious effects have not yet been developed. In addition, commercially available drugs may cause numerous side effects such as hepatotoxicity, insomnia, hypertension, nausea, *etc.* Therefore, there is a strong and urgent need to develop therapeutic agents capable of effectively treating cognitive impairment-related diseases without causing side effects.

### DESCRIPTION OF EMBODIMENTS

### TECHNICAL PROBLEM

As a result of intensive research to develop substances having therapeutic effects on cognitive impairment-related diseases such as dementia, Alzheimer's disease, and memory impairment, the present inventors have confirmed that mumefural has excellent therapeutic effects on brain damage, thereby completing the present invention.

### SOLUTION TO PROBLEM

An object of the present invention is to provide a pharmaceutical composition for preventing or treating a cognitive impairment-related disease, which includes mumefural or a pharmaceutically acceptable salt thereof as an active ingredient.

Another object of the present invention is to provide a use of the composition including mumefural or a pharmaceutically acceptable salt thereof as an active ingredient for preventing or treating a cognitive impairment-related disease.

Still another object of the present invention is to provide a method of treating a cognitive impairment-related disease, the method including administering the pharmaceutical composition to an individual suspected of having a cognitive impairment-related disease.

Still another object of the present invention is to provide a health functional food composition for preventing or alleviating a cognitive impairment-related disease, which includes mumefural as an active ingredient.

Still another object of the present invention is to provide a health functional food composition for enhancing learning ability, cognitive function, or memory, which includes mumefural as an active ingredient.

Still another object of the present invention is to provide a feed composition for preventing or alleviating a cognitive impairment-related disease, which includes mumefural as an active ingredient.

### ADVANTAGEOUS EFFECTS OF DISCLOSURE

Due to excellent effects of the mumefural of the present invention on normalizing damage to the brain (*i.e*., basal forebrain, white matter, hippocampus, *etc*.) and excellent effects thereof on enhancing memory, the mumefural of the present invention may be effectively used to treat neurodegenerative diseases (*i.e*., dementia, Alzheimer's disease, *etc*.) and cognitive impairment caused by brain damage (*i.e*., memory impairment), and to enhance learning ability, cognitive function, and memory.

### BRIEF DESCRIPTION OF DRAWINGS

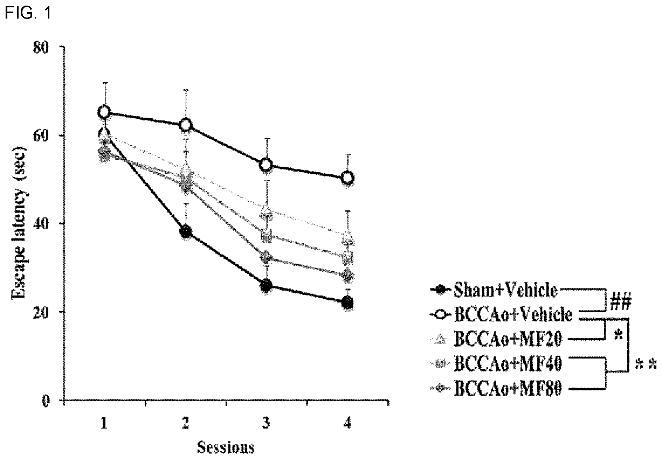

FIG. 1 shows effects of mumefural on enhancing spatial memory, demonstrating escape latency with respect to sessions. In particular, Sham+Vehicle (saline) is a sham-operated group as a normal control; BCCAo+Vehicle (saline) is a brain-damaged group treated with saline as a negative control; BCCAo+MF20 is a brain-damaged group treated with 20 mg/kg of mumefural as an experimental group; BCCAo+MF40 is a brain-damaged group treated with 40 mg/kg of mumefural as an experimental group; and BCCAo+MF80 is a brain-damaged group treated with 80 mg/kg of mumefural as an experimental group, and the descriptions of the categories are applied to the following drawings.
FIG. 2 shows effects of mumefural on enhancing spatial recognition memory, demonstrating time first arriving at a test platform (time to 1^{st} platform entry; sec).
FIG. 3 shows biosafety of mumefural, demonstrating average swimming speed (m/sec) with respect to sessions.
FIG. 4A shows effects of mumefural on normalization of damaged basal forebrain (normalization of ChAT-positive cholinergic cells), demonstrating immunohistostaining results of ChAT protein.
FIG. 4B shows quantified immunohistostaining results of ChAT-positive cholinergic cells on normalization according to FIG. 4A.
FIG. 5A shows effects of mumefural on normalization of the cholinergic system the in basal forebrain, demonstrating western blot analysis results of ChAT, VAChT, and AChE proteins.
FIG. 5B shows quantified western blot analysis results of ChAT, VAChT, and AChE proteins according to FIG. 5A.
FIG. 6 shows effects of mumefural on normalization of the cholinergic system in the basal forebrain, demonstrating enzyme-linked immunosorbent assay results of AChE activity.
FIG. 7A shows effects of mumefural on normalization of the cholinergic system in the hippocampus, demonstrating western blot analysis results of ChAT, VAChT, and AChE proteins.
FIG. 7B shows quantified western blot analysis results of ChAT, VAChT, and AChE proteins according to FIG. 7A.
FIG. 8 shows effects of mumefural on normalization of the cholinergic system in the hippocampus, demonstrating enzyme-linked immunosorbent assay results of AChE activity.
FIG. 9A shows effects of mumefural on normalization of degraded myelin in white matter and the hippocampus, demonstrating immunohistostaining results of MBP protein.
FIG. 9B shows quantified immunohistostaining results of MBP protein according to FIG. 9A.
FIG. 10 shows effects of mumefural on normalization of MBP expression in the hippocampus (inhibition on exfoliation of MBP), demonstrating western blot analysis results of MBP.
FIG. 11A shows effects of mumefural on normalization of synaptic markers (increase in expression of PSD-95 and synaptophysin-1 proteins) in the hippocampus, demonstrating western blot analysis results of PSD-95 and synaptophysin-1 proteins.
FIG. 11B shows quantified western blot analysis results of PSD-95 and synaptophysin-1 proteins according to FIG. 11A.
FIG. 12A shows effects of mumefural on normalization of amount of post-synaptic receptors (increase in expression of NMDAR2A and NMDAR2B proteins) in the hippocampus, demonstrating western blot analysis results of NMDAR2A and NMDAR2B proteins.
FIG. 12B shows quantified western blot analysis results of NMDAR2A and NMDAR2B proteins according to FIG. 12A.
FIG. 13A shows effects of mumefural on normalization of neurotransmitter secretion substances (increase in expression of phospho-CAMKII protein) in the hippocampus, demonstrating western blot analysis results of phospho-CAMKII protein.
FIG. 13B shows quantified western blot analysis results of phospho-CAMKII protein according to FIG. 13A.
FIG. 14A shows effects of mumefural on normalization of a memory-related factor (increase in expression of BDNF and phospho-CREB proteins) in the hippocampus, demonstrating western blot analysis results of BDNF and phospho-CREB proteins.
FIG. 14B shows quantified western blot analysis results of BDNF and phospho-CREB proteins according to FIG. 14A.
FIG. 15A shows effects of mumefural on normalization of damaged hippocampus (inhibition of microglia), demonstrating immunohistostaining analysis results of ionized calcium-binding adaptor molecule (lba-1). In particular, CA 1 means Cornus Ammonis 1 of the hippocampus, CA 3 means Cornus Ammonis 3 of the hippocampus, and DG means dentate gyrus of the hippocampus.
FIG. 15B shows quantified immunohistostaining results according to FIG. 15A and demonstrates the number of Iba-1 positive cells.
FIG. 15C shows effects of mumefural on normalization of damaged white matter (inhibition of microglia), demonstrating immunohistostaining analysis results of Iba-1. In particular, white matter lesion means corpus callosum, fimbria hippocampi, and optic tract.
FIG. 15D shows quantified immunohistostaining results according to FIG. 15C and demonstrates the number of Iba-1 positive cells.
FIG. 16A shows effects of mumefural on normalization of damaged hippocampus (inhibition of astrocytes), demonstrating immunohistostaining analysis results of Glial fibrillary acidic protein (GFAP). In particular, CA 1 means Cornus Ammonis 1 of the hippocampus, CA 3 means Cornus Ammonis 3 of the hippocampus, and DG means dentate gyrus of the hippocampus.
FIG. 16B shows quantified immunohistostaining analysis results according to FIG. 16A and demonstrates the number of GFAP positive cells.
FIG. 16C shows effects of mumefural on normalization of damaged white matter (inhibition of astrocytes), demonstrating immunohistostaining analysis results of GFAP. In particular, white matter lesion means corpus callosum, fimbria hippocampi, and optic tract.
FIG. 16D shows quantified immunohistostaining results according to FIG. 16C and demonstrates the number of GFAP positive cells.
FIG. 17A shows effects of mumefural on normalization of neurological inflammation (decrease in expression of P2X7R, TLR4, MyD88, NLRP3, caspase1, IL-1β, and IL-18 proteins) in the hippocampus, demonstrating western blot analysis results of P2X7R, TLR4, MyD88, NLRP3, caspase1, IL-1β, and IL-18 proteins.
FIG. 17B shows quantified western blot analysis results of P2X7R, TLR4, MyD88, NLRP3, caspase1, IL-1β, and IL-18 proteins according to FIG. 17A.
FIG. 18A shows effects of mumefural on normalization of a neurological inflammation-related transcription factor (decrease in expression of phospho-STAT3 protein) in the hippocampus, demonstrating western blot analysis results of phospho-STAT3 protein.
FIG. 18B shows quantified western blot analysis results of phospho-STAT3 protein according to FIG. 18A.
FIG. 19A shows effects of mumefural on normalization of neurological inflammation signaling (decrease in expression of NF-κB protein), demonstrating western blot analysis results of NF-κB (p65, p50) protein.
FIG. 19B shows quantified western blot analysis results of NF-κB (p65, p50) protein in the hippocampus according to FIG. 19A.
FIG. 20 shows effects of mumefural on normalization of neurological inflammation cytokines (decrease in cytokines IL-1β and IL-18) in the hippocampus, demonstrating enzyme-linked immunosorbent assay results of cytokines IL-1β and IL-18.

### BEST MODE

An aspect of the present invention to achieve the above objects provides a pharmaceutical composition for preventing or treating a cognitive impairment-related disease, which includes mumefural or a pharmaceutically acceptable salt thereof as an active ingredient.

Another aspect of the present invention provides a use of the composition, which includes mumefural or a pharmaceutically acceptable salt thereof as an active ingredient, for preventing or treating a cognitive impairment-related disease.

In the present invention, it is confirmed that mumefural has effects on enhancement of spatial recognition memory, normalization of the cholinergic system, normalization of degraded myelin, normalization of synaptic markers, normalization of hippocampus damage, normalization of gliosis, and decrease in neurological inflammation, which are well known as treatment mechanisms of cognitive impairment-related diseases, and thus, it is confirmed that mumefural may be effectively used for prevention, alleviation, or treatment of cognitive impairment-related diseases.

As used herein, the term "mumefural" refers to a compound having a structure represented by Formula 1 below. Although mumefural is known to have pharmacological characteristics such as antiviral effects, preventive, therapeutic, and alleviative effects thereof on cognitive impairment-related diseases such as vascular dementia, Alzheimer's dementia, Alzheimer's disease, and memory impairment, these have not been reported and were revealed by the present inventors for the first time.

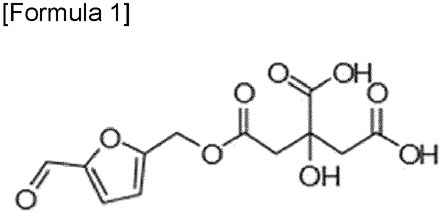

The methods of obtaining mumefural described above are not particularly limited, and mumefural may be chemically synthesized or isolated via any method known in the art, or commercially available mumefural may be used.

In addition, the compound may be present in a solvated or unsolvated form in a crystal or amorphous form, and these physical forms are included within the scope of the present invention.

As used herein, the term "cognitive impairment-related disease" refers to a disease caused by decline in cognitive function (cognitive ability), such as ability to remember, spatiotemporal recognition ability, decision-making judgment, language ability, and computation ability, due to brain damage. Specific examples thereof may be dementia, Alzheimer's disease, or memory impairment. More specifically, the dementia may be vascular dementia or Alzheimer's dementia, but the disease is not limited thereto as long as cognitive function disorders are apparent. Although exact pathogenesis and causes of cognitive impairment-related diseases have not been reported, decreases in synaptic markers, amount of post-synaptic receptors, and memory-related factor, and neurological inflammation caused by damage to brain such as basal forebrain, white matter, and hippocampus have been known to date as causes of the cognitive impairment-related diseases.

Dementia is a condition in which cognitive function is impaired due to various causes, and there are various sub-diseases depending on the causes. Specific examples thereof include senile dementia, Alzheimer's disease, vascular dementia, dementia with Lewy bodies, frontotemporal dementia, Parkinson's disease dementia, Huntington's disease dementia, *etc.* The cognitive impairment-related disease of the present invention includes all of the sub-diseases.

Alzheimer's disease, which is the most common degenerative brain disease causing dementia, develops slowly and gradually worsens cognitive function. Symptoms of cognitive impairment such as memory loss, declined language ability, decline in spatiotemporal recognition ability, impaired decision-making judgment, inability to perform most common activities of daily living, walking disruptions, and behavioral disorders are observed.

Memory impairment is a pathological condition of having difficulty or inability to remember objects or recall past experiences, and examples thereof may include forgetfulness, blackout, short-term memory loss, long-term memory loss, and transient memory impairment.

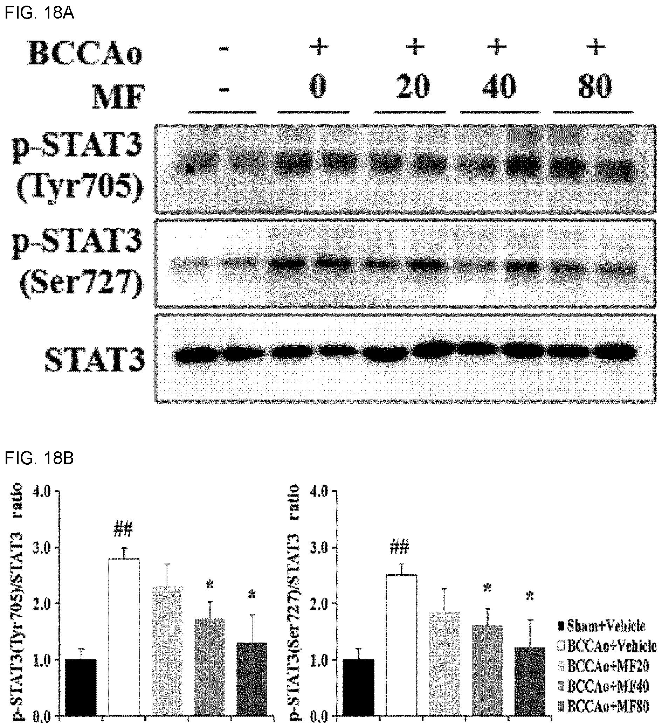

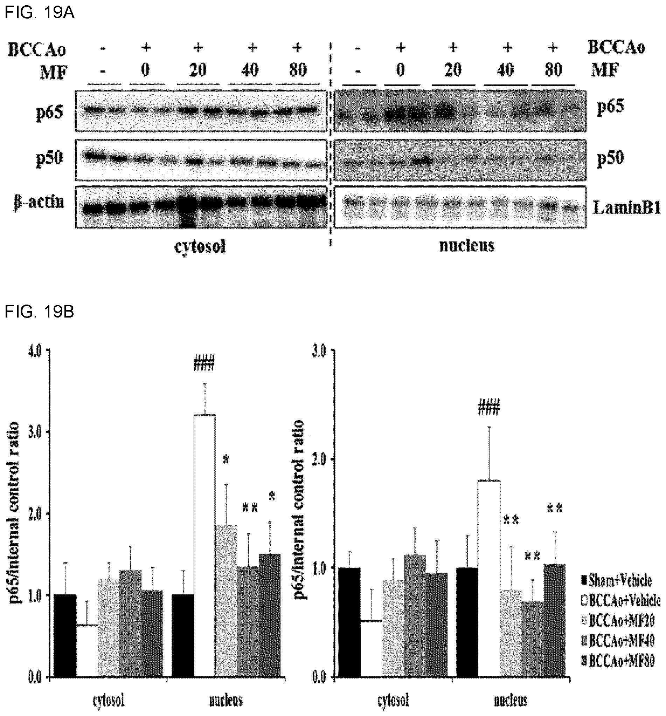

In an embodiment of the present invention, it was confirmed that mumefural has effects on enhancing spatial recognition memory, normalizing damage to the basal forebrain, white matter, the hippocampus, or the like, increasing synaptic markers, amount of post-synaptic receptors, and memory-related factor, or the like, and reducing neurological inflammation without adversely affecting motor ability in a dementia-mimic animal model. In addition, the effects were confirmed to be similar or superior to those of a normal control in which dementia is not induced (FIGS. 1 to 20).

This indicates that mumefural may be used efficiently and safely to prevent or treat a cognitive impairment-related disease such as memory impairment, Alzheimer's disease, dementia, *etc.*

As used herein, the term "prevention" refers to any action to inhibit or delay the cognitive impairment-related disease by administering a composition including mumefural or a pharmaceutically acceptable salt thereof.

As used herein, the term "treatment" refers to any action to ameliorate or beneficially change symptoms associated with the cognitive impairment-related disease by administering a composition including mumefural or a pharmaceutically acceptable salt thereof.

As used herein, the term "pharmaceutically acceptable salt" refers to a salt available in preparation of drugs among salts in which a cation and an anion are bound by electrostatic attraction. In general, a metal salt, a salt with an organic base, a salt with an inorganic acid, a salt with an organic acid, a salt with a basic or acidic amino acid, or the like may be used. For example, the metal salt may be a salt of an alkali metal (sodium salt, potassium salt, or the like), a salt of an alkali earth metal (calcium salt, magnesium salt, barium salt, or the like), or an aluminum salt; the salt with an organic base may be a salt with triethylamine, pyridine, picoline, 2,6-lutidine, ethanolamine, diethanolamine, triethanolamine, cyclohexylamine, dicyclohexylamine, *N*,*N*-dibenzylethylenediamine, or the like; the salt with an inorganic acid may be a salt with hydrochloric acid, hydrobromic acid, nitric acid, sulfuric acid, phosphoric acid, or the like; the salt with an organic acid may be a salt with formic acid, acetic acid, trifuloroacetic acid, phthalic acid, fumaric acid, oxalic acid, tartaric acid, maleic acid, citric acid, succinic acid, methanesulfonic acid, benzenesulfonic acid, *p*-toluenesulfonic acid, or the like; the salt with a basic amino acid may be a salt with arginine, lysine, ornithine, or the like; and the salt with an acidic amino acid may be aspartic acid, glutamic acid, or the like.

The pharmaceutical composition of the present invention includes mumefural or a pharmaceutically acceptable salt in an amount of 0.01 wt% to 80 wt%, specifically, 0.01 wt% to 70 wt%, more specifically 0.01 wt% to 60 wt% based on a total weight of the composition, but the amount is not limited as long as preventive or therapeutic effects on the cognitive impairment-related disease are obtained.

In addition, the pharmaceutical composition may further include a pharmaceutically acceptable carrier, excipient, or diluent commonly used in preparation of pharmaceutical compositions, and the carrier may include a carrier which is not naturally occurring. Specific examples of the carrier, excipient, and diluent may be lactose, dextrose, sucrose, sorbitol, mannitol, xylitol, erythritol, maltitol, starch, Acacia rubber, alginate, gelatin, calcium phosphate, calcium silicate, cellulose, methyl cellulose, amorphous cellulose, polyvinyl pyrrolidone, water, methylhydroxy benzoate, propylhydroxy benzoate, talc, magnesium stearate, or mineral oil, but these are not limited thereto.

In addition, the pharmaceutical composition may be used in a formulation selected from the group consisting of a tablet, a pill, powder, granules, a capsule, a suspension, a solution for internal use, an emulsion, a syrup, a sterilized aqueous solution, a non-aqueous solvent, a lyophilized preparation, and a suppository according to the conventional methods for oral or parenteral administrations. When prepared into formulations, a diluent or excipient such as a filler, a bulking agent, a binder, a humectant, a disintegrating agent, or a surfactant commonly available in the art may be used. A solid formulation for oral administration may be a tablet, a pill, powder, granules, a capsule, or the like, and the solid formulation may include at least one excipient, *e.g*., starch, calcium carbonate, sucrose, lactose, or gelatin. Additionally, in addition to such excipients, a lubricating agent such as magnesium stearate or talc may be used. A liquid formulation for oral administration may be a suspension, a solution for internal use, an emulsion, a syrup, or the like, and the liquid formulation may include various excipients such as a humectant, a sweetener, an aromatic, or a preservative in addition to a conventional diluent such as water or liquid paraffin. A formulation for parental administration may include a sterile aqueous solution, a non-aqueous solvent, a suspension, an emulsion, a lyophilizate, a suppository, or the like. The non-aqueous solvent and the suspension may include propylene glycol, polyethylene glycol, vegetable oil such as olive oil, or an injectable ester such as ethyloleate. A base for the suppository may be Witepsol, Macrogol, tween 61, cacao butter, laurin butter, glycerogelatin, or the like, but is not limited thereto.

Another aspect of the present invention provides a method of treating a cognitive impairment-related disease, including administering the composition to an individual suspected of having a cognitive impairment-related disease.

In particular, definitions of the "cognitive impairment-related disease" and "treatment" are as described above.

As used herein, the term "administration" refers to introduction of the pharmaceutical composition to an individual in an appropriate manner.

As used herein, the term "individual" refers to any animal including humans, rats, mice, and livestock who have a cognitive impairment-related disease which has already developed or is likely to develop. Specific examples thereof may be mammals including humans.

The pharmaceutical composition of the present invention may be administered in a pharmaceutically effective amount.

The term "pharmaceutically effective amount" refers to an amount sufficient for treating diseases at a reasonable benefit/risk ratio applicable to medical treatment, and a level of an effective dose may be determined based on factors including type of a subject, severity of disease, age, gender, drug activity, drug sensitivity, administration time, administration route, and excretion rate, treatment duration, drug(s) to be concurrently used in combination, and other factors well known in the medical field.

The pharmaceutical composition may be administered as an individual therapeutic agent, in combination with other therapeutic agents, or sequentially or simultaneously with a conventional therapeutic agent(s), and may be administered once or multiple times. It is important to administer an amount to obtain the maximum effect with a minimum amount without adverse effects in consideration of the factors described above, and the amount may easily be determined by one of ordinary skill in the art.

In addition, the pharmaceutical composition of the present invention may be administered orally or parenterally (*e.g*., intravenously, subcutaneously, intraperitoneally, or topically) according to a desired method. A dosage may be appropriately selected by one of ordinary skill in the art, although the dosage varies according to status and body weight of a patient, severity of disease, formulation of drug, administration route, and administration time. Specifically, a daily dosage of the pharmaceutical composition may generally be from 0.001 mg/kg to 1,000 mg/kg, more specifically from 0.05 mg/kg to 200 mg/kg, and most specifically from 0.1 mg/kg to 100 mg/kg once to several times per day. However, a preferable dosage may be appropriately selected by one of ordinary skill in the art based on the status and body weight of the individual, severity of disease, formulation of drug, and administration route and time.

Still another aspect of the present invention provides a health functional food composition for preventing or alleviating a cognitive impairment-related disease, which includes mumefural or a physiologically acceptable salt thereof as an active ingredient.

Still another aspect of the present invention provides a health functional food composition for enhancing learning ability, cognitive function, or memory, which includes mumefural or a physiologically acceptable salt thereof as an active ingredient.

In particular, definitions of the "mumefural", "cognitive impairment-related disease", and "prevention" are as described above.

As used herein, the term "learning" refers to the ability or behavior to perceive and change one's own behavior and includes spatial perception, cognition, concentration, *etc.*

As used herein, the term "cognitive function" refers to the ability to obtain, maintain, and utilize information and includes cognitive ability such as ability to remember, spatiotemporal recognition ability, decision-making judgment, language ability, and computation ability.

As used herein, the term "memory" or "ability to remember" refers to ability to encode and store new information obtained from the surrounding environment, learned experience, and knowledge in a particular area of the brain and retrieve stored data.

As used herein, the term "physiologically acceptable salt" refers to a generally available salt which is physiologically acceptable and which is capable of obtaining desired effects when administered to living organisms without causing allergic reactions such as gastrointestinal disorders and dizziness or similar reactions.

As used herein, the term "alleviation" refers to any action to at least decrease parameters, *e.g*., the degree of symptoms related to a condition to be treated by administering the composition including mumefural.

Due to excellent therapeutic effects on brain damage and enhancing effects on memory, the mumefural according to the present invention may be included in health functional food compositions to prevent or alleviate cognitive impairment-related diseases or to enhance learning ability, cognitive function, or memory. Since the health functional food composition may be consumed on a daily basis, excellent preventive or alleviative effects on a cognitive impairment-related disease or excellent enhancing effects on learning, cognitive function, or memory may be expected.

As used herein, the term "health functional food" means food manufactured and processed with functional raw materials or ingredients beneficial to human health under Health Functional Food Act No. 6727, and the term "functionality" means controlling nutrients for the structure or functions of the human body or providing beneficial effects for health purposes, such as physiological effects. Meanwhile, health food refers to food having an effect on maintaining or promoting health conditions compared to general foods, and health supplement food refers to food for health supplementation. In some cases, the terms "health functional food", "health food", and "health supplement food" may be used interchangeably.

The mumefural of the present invention may be added as it is or used in combination with other foods or food components, and may be properly used according to a common method.

The health functional food composition of the present invention may be prepared by a method commonly used in the art, and raw materials and ingredients typically used in the art may be added thereto for preparation of the health functional food. Specifically, the health functional food composition may further include a physiologically acceptable carrier, and the type of carrier is not particularly limited, and any carrier may be used as long as it is commonly used in the art.

In addition, the health functional food composition may include food additives such as a preservative, a disinfectant, an antioxidant, a coloring agent, a color-developing agent, a bleaching agent, a seasoning, a flavor, a swelling agent, a fortifier, an emulsifier, a thickener, a film-forming agent, a gum base agent, an antifoaming agent, a solvent, and an improver. These additives may be selected and used in an appropriate amount according to the type of food.

In addition, the health functional food may be prepared into any formulation that is regarded as food, without limitations. The health functional food composition of the present invention may be prepared into various formulations. Due to advantages over general drugs in that the food composition is free of side effects which may occur upon long-term intake of drugs because it is manufactured using food ingredients and has high portability, the health functional food composition may be ingested as an aid for promoting preventive and alleviative effects on cognitive impairment-related diseases.

The mumefural of the present invention may be included in the health function food composition in various percentages by weight (wt%) as long as preventive or alleviative effects on cognitive impairment-related diseases or enhancing effects on learning ability, cognitive function, or memory are obtained. Specifically, the mumefural may be included in an amount of 0.00001 wt% to 100 wt% or 0.01 wt% to 80 wt% based on a total weight of the health functional food composition, but is not limited thereto. When prolonged intake is intended for the purpose of health and hygiene, the amount may be below the above range. In addition, since there is no safety problem, the active ingredient may be used in an amount above the range.

Still another aspect of the present invention provides a feed composition for preventing or alleviating a cognitive impairment-related disease, which includes mumefural or a physiologically acceptable salt thereof as an active ingredient.

In particular, definitions of the "mumefural", "physiologically acceptable salt", "cognitive impairment-related disease", "prevention", and "alleviation" are as described above.

As used herein, the term "feed" refers to any natural or artificial diet, a meal, or components thereof for animals to eat, ingest, and digest.

The types of feed are not particularly limited, and any feeds commonly available in the art may be used. Non-limiting examples of the feed include: vegetable feeds such as grains, root plants, food processing by-products, algae, fibers, pharmaceutical by-products, fat and oils, starches, *Cucurbitaceae* vegetables, or grain by-products; and animal feed such as proteins, inorganic substances, fat and oils, minerals, single-cell proteins, animal plankton, or foods. These feeds may be used alone or in a combination of at least two thereof.

The feed composition of the present invention may be prepared in various formulations well known in the art. In addition, the feed composition may further include substances exhibiting various effects such as supplementing nutritional elements, preventing weight loss, enhancing digestion of fibers within the feed, improving milk quality, preventing reproductive disorders, improving pregnancy rate, and preventing high-temperature stress during summer. Examples thereof further include: mineral formulations including sodium hydrogen carbonate, bentonite, magnesium oxide, and complex minerals, and trace minerals such as zinc, copper, cobalt, and selenium; vitamins such as carotene, vitamins A, D, and E, nicotinic acid, and vitamin B complex; amino acid protective agents such as methionine and lysine; fatty acid protective agents such as fatty acid calcium; and live bacterial cells and yeast such as probiotics (lactic acid bacteria), yeast culture, and fungus culture.

### MODE OF DISCLOSURE

Hereinafter, the present invention will be described in more detail with reference to the following examples. However, these examples are for illustrative purposes only and are not intended to limit the scope of the present invention.

### Example 1. Construction of Dementia-mimic Animal Model and Establishment of Oral Administration Method

### Example 1-1. Method of Constructing Dementia-mimic Animal Model

In order to investigate inhibitory effects of mumefural (MF) on vascular dementia or memory decline progression, first, a dementia-mimic animal model was constructed.

Meanwhile, the mumefural (MF) used in the present invention was purchased from U-Chem (Seoul) (purity: 95% or more) and used in experiments after verification by NMR and Mass Analysis.

Specifically, 12-week-old Wister rats (350 g to 380 g) were used for the experiments. After obtaining the experimental animals, appearance was visually inspected and then normal symptoms were observed during a 7-day acclimation period. Healthy animals were selected therefrom and assigned to groups by a random method according to body weight ranges, and then experiments were performed. During the acclimation and experiment, a constant breeding environment was maintained under the conditions of a temperature of 23±3°C, a relative humidity of 50±10%, a ventilation frequency of 12 times to 16 times per hour, a 12-hour light-dark cycle (light on at 7:00, light off at 19:00), and an intensity of illumination of 150 Lx to 300 Lx. Experiments were performed using sterilized instruments, and the rats were allowed free access to tap water and a solid feed (PMI nutrition, USA) for 24 hours.

In order to construct a vascular dementia-mimic animal model, chronic cerebral hypoperfusion was induced by bilateral common carotid artery occlusion (2VO, hereinafter referred to as "brain damage (BCCAo)") (Wakita *et al*., 1994). The rats were anesthetized with 4% isoflurane, and the anesthesia was maintained by 1.5% isoflurane during the operation. The central neck was incised to expose the bilateral common carotid artery carefully so as not to cause damage to the vagal nerve. Ligation was performed twice with No. 3 silk, thereby constructing an animal model in which dementia was induced by brain damage.

### Example 1-2. Oral Administration Method of Mumefural

After 21 days from the BCCAo operation, the Wistar rats were orally administered with mumefural for 42 days. The rats were divided into 5 groups listed in Table 1 below, and controls were orally administered with saline. Different concentrations of mumefural were set in the oral administration as low concentration (20 mg/kg), medium concentration (40 mg/kg), and high concentration (80 mg/kg).

**Table 1**

| | Group | Cognitive function verification (number) | Neurobiological verification (number) |
|---|---|---|---|
| Normal control | Sham-operated group (Sham) + Vehicle (saline) | 12 | 12 |
| Negative control | Brain-damaged group (BCCAo) + Vehicle (saline) | 10 | 10 |
| Experime ntal group | Brain-damaged group (BCCAo) + mumefural (20 mg/kg) | 11 | 11 |
| | Brain-damaged group (BCCAo) + mumefural (40 mg/kg) | 11 | 11 |
| | Brain-damaged group (BCCAo) + mumefural (80 mg/kg) | 5 | 5 |

### Example 2. Confirmation of Effects of Mumefural on Enhancing Spatial memory

In order to investigate effects of mumefural on enhancing spatial memory in the dementia-mimic animal model, a water maze test was performed after administering mumefural in the same manner as in Example 1 above.

Specifically, a circular water tank (diameter: 180 cm, height: 58 cm) was filled with water at a temperature of 26±2°C up to 2 cm above a marked platform, screen walls were set up on all sides of the water tank to prevent light from coming through, and a pigment was added thereto to make the water opaque. In addition, labels were attached to given positions (diameter: 12 cm, height: 33.5 cm) of the screen walls to give clues for the rats to search for the marked platform. Spatial memory training was performed 4 times per day for 8 days every morning (from 9:00 a.m.) once per day 8 times in total. Time taken for the rats to find the hidden platform and escape latency thereof were measured and regarded as markers of memory, and reduced time of every group was compared every day. Thereafter, a water maze test was performed after 8 days from the training. In this test, time taken for the rats to first find the hidden platform was measured and used as a marker of memory. That is, a shorter time is regarded as more improved recognition ability.

As a result, as shown in FIG. 1, it was confirmed that the latency to find the hidden platform increased in the experimental groups treated with mumefural compared with the negative control as the numbers of administration of mumefural and training increased, and the experimental groups treated with 20 mg/kg, 40 mg/kg, and 80 mg/kg of mumefural exhibited high latency similar to that of the normal control.

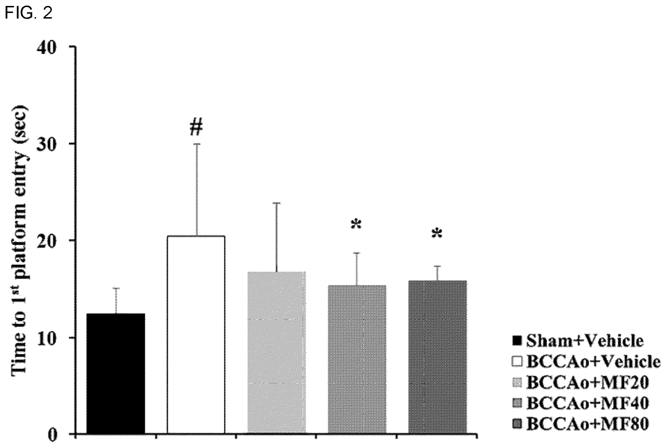

In addition, as shown in FIG. 2, it was confirmed that the experimental groups treated with mumefural demonstrated reduced time to first arrive at the hidden platform when compared to the negative control treated with saline, and in particular, the experimental groups treated with 40 mg/kg and 80 mg/kg of mumefural arrived within a short time similar to that of the normal control.

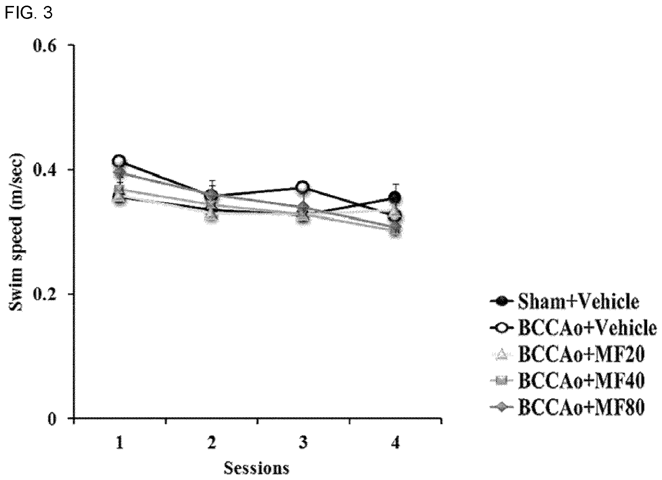

Furthermore, as shown in FIG. 3, it was confirmed that there was no difference in average swim speeds among the groups.

Based on the results, mumefural effectively normalizes the spatial memory reduced by brain damage in the dementia-mimic rats and does not exhibit side effects such as motor disturbance. Therefore, it may be confirmed that mumefural may be effectively and safely used in prevention, treatment, and the like of cognitive impairment-related diseases such as memory impairment, Alzheimer's disease, and dementia.

### Example 3. Confirmation of Effects of Mumefural on Normalization of Cholinergic System

In order to investigate effects of mumefural on normalization of the cholinergic system in the dementia-mimic animal model, mumefural was administered in the same manner as in Example 1, and then the number of cholinergic neurons of the basal forebrain was measured.

Specifically, the brain was excised from each of the dementia-mimic rats after administration of mumefural was completed and sectioned into slices with a thickness of 40 µm. Subsequently, the cholinergic neurons of the basal forebrain were detected using choline acetyltransferase (ChAT) protein, which is known to be expressed in cholinergic neurons. The slices were stained by immunohistostaining using an anti-ChAT antibody as a primary antibody and a donkey anti-goat antibody as a secondary antibody, and anti-ChAT positive cells were detected.

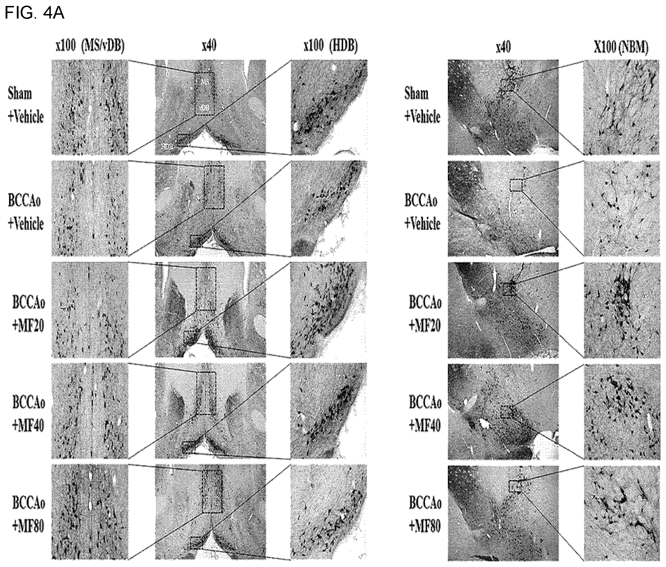

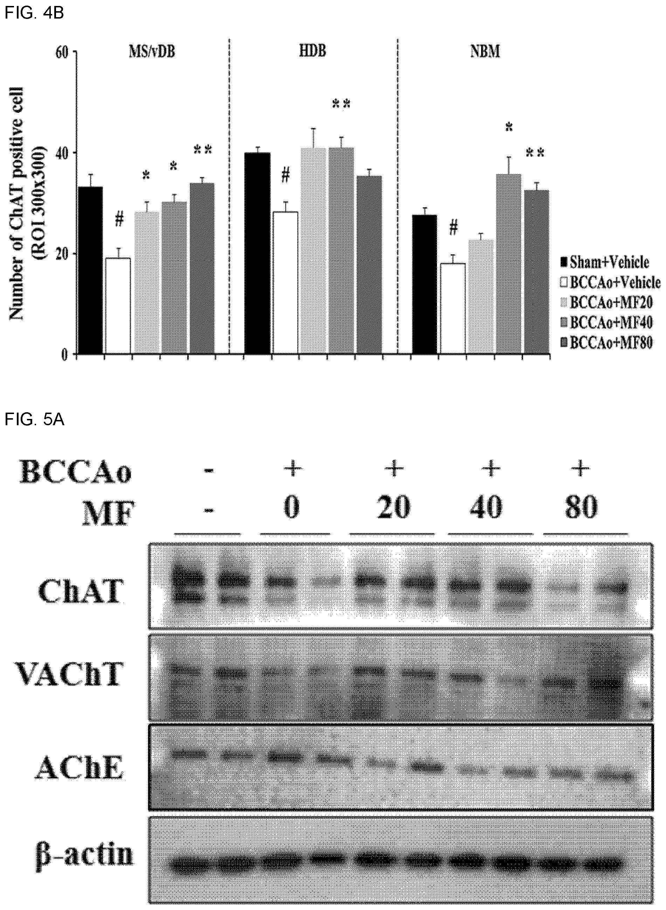

As a result, as shown in FIGS. 4A and 4B, it was confirmed that the experimental groups treated with mumefural had a significantly increased number of cells positive for the anti-ChAT antibody in the basal forebrain when compared with the negative control, and it was confirmed that the experimental groups treated with 20 mg/kg, 40 mg/kg, and 80 mg/kg of mumefural had similar effects to those of the normal control.

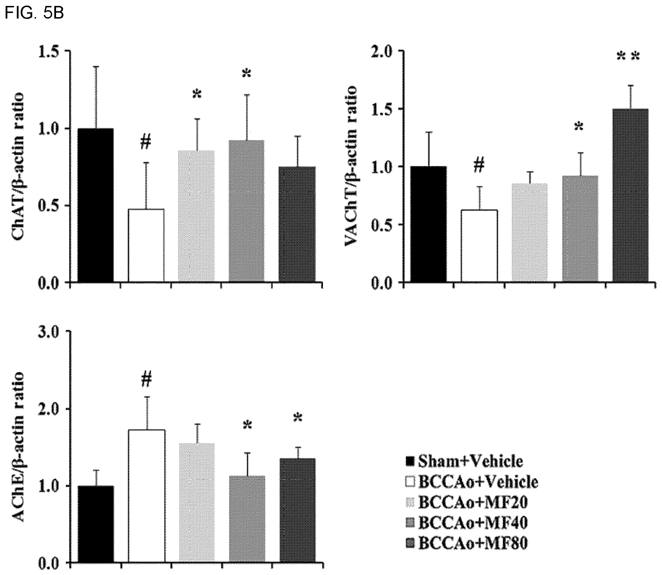

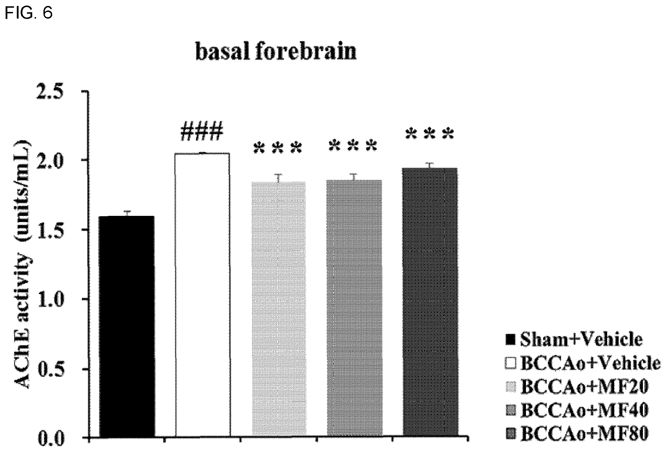

In addition, when compared with the negative control treated with saline, the experimental groups treated with mumefural exhibited increased expression levels of ChAT and VAChT proteins in the basal forebrain and reduced expression levels of AChE protein, as shown in FIGS. 5A and 5B, and reduced activity of AChE, as shown in FIG. 6, and it was confirmed that the experimental groups treated with 20 mg/kg, 40 mg/kg, and 80 mg/kg of mumefural had similar effects to those of the normal control.

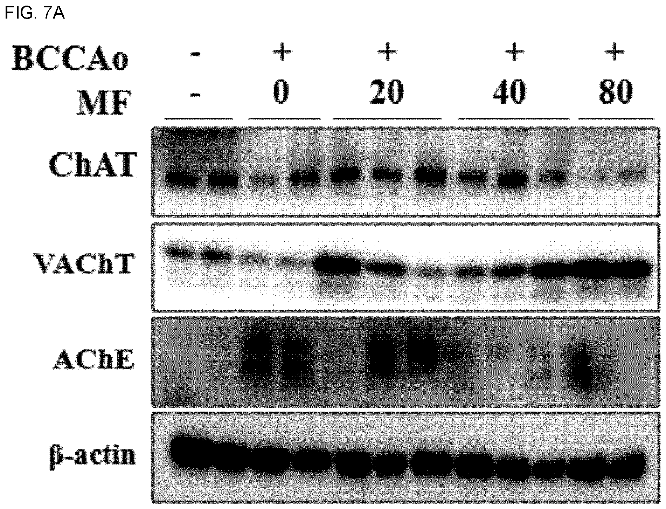

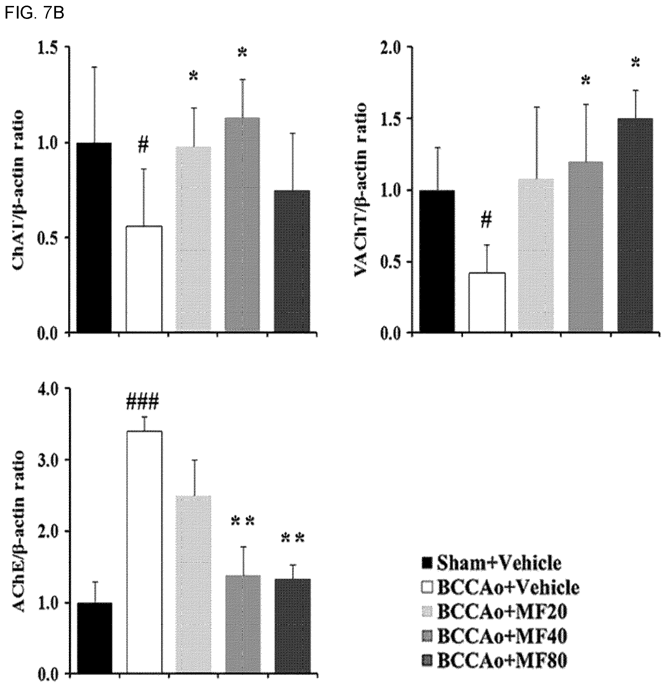

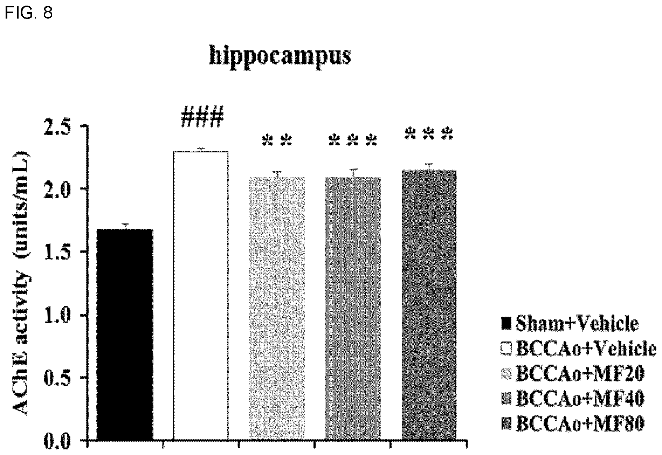

In addition, it was confirmed that the experimental groups treated with mumefural showed increased expression levels of ChAT and VAChT proteins and decreased expression levels of AChE protein in the hippocampus when compared with the negative control, as shown in FIGS. 7A and 7B, and decreased activity of AChE, as shown in FIG. 8, and it was confirmed that the experimental groups treated with 40 mg/kg and 80 mg/kg of mumefural had similar effects to those of the normal control.

Based on the results, mumefural not only normalizes cholinergic neurons in the basal forebrain by increasing the number of cholinergic neurons that had been reduced by brain damage but also reduces the activity of AChE in the hippocampus in the dementia-mimic rats, and thus it was confirmed that mumefural may be effectively and safely used in prevention, treatment, and the like of the cognitive impairment-related disease such as memory impairment, Alzheimer's disease, and dementia.

### Example 4. Confirmation of Effects of Mumefural on Normalization of Degraded Myelin

In order to investigate the effects on normalization of degraded myelin in the dementia-mimic animal model, mumefural was administered in the same manner as in Example 1 above, and then degradation degrees of oligodendrocytes was analyzed in the hippocampus, fornix, medial septum, corpus callosum, and fimbria hippocampi.

Specifically, the brain was excised from each of the dementia-mimic rats after administration of mumefural was completed and sectioned into slices with a thickness of 40 µm. Subsequently, oligodendrocytes in the hippocampus, white matter fornix, medial septum, corpus callosum, and fimbria hippocampi were detected using myelin basic protein (MBP) that is expressed in the myelin sheath of axons by oligodendrocytes. The slices were stained by immunohistostaining using anti-MBP antibody as a primary antibody and horse anti-mouse antibody as a secondary antibody, and anti-MBP positive cell responses were detected.

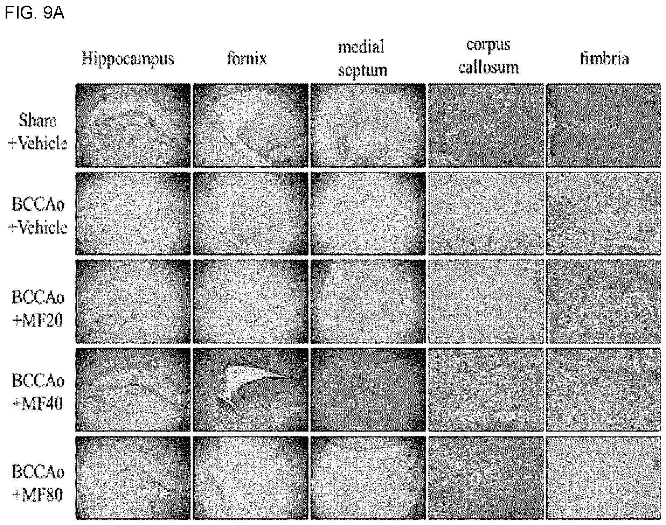

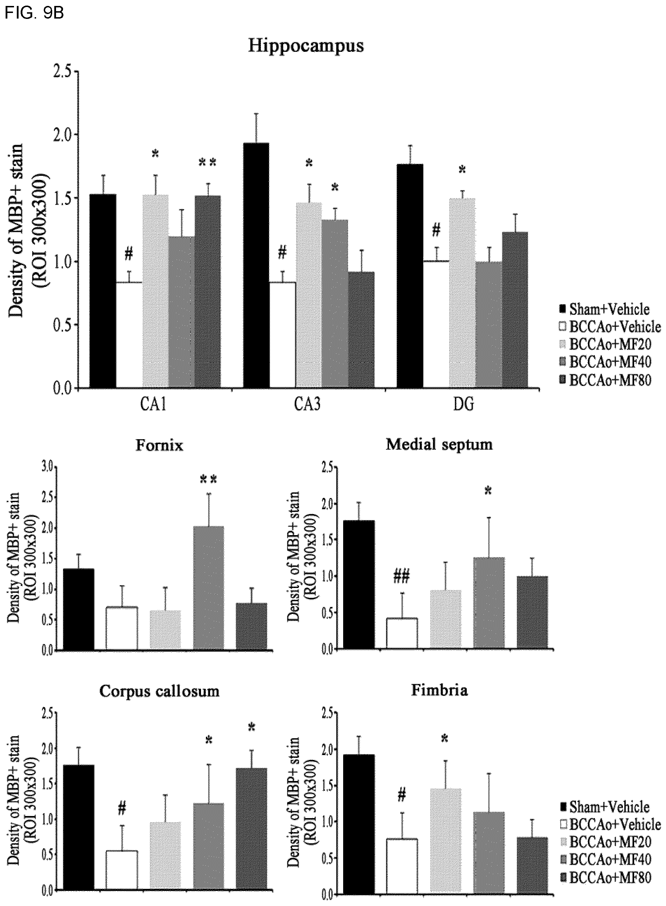

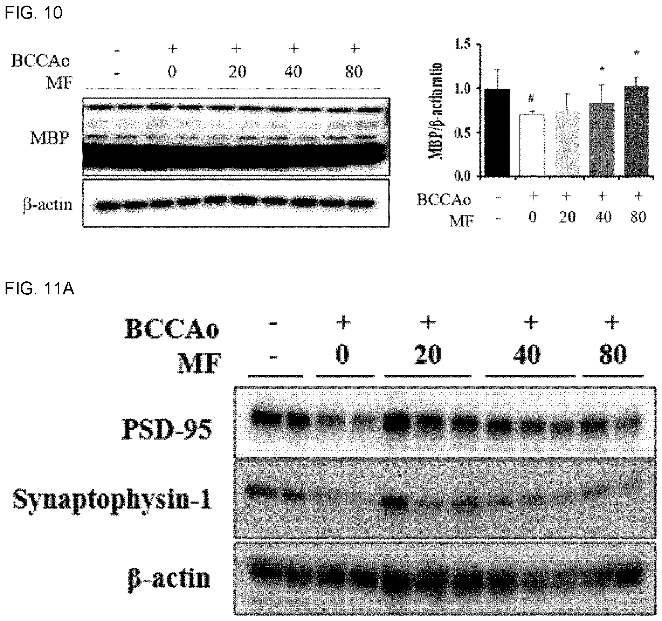

As a result, it was confirmed that the experimental groups treated with mumefural showed decreased myelin degradation, as shown in FIGS. 9A and 9B, and increased density of the MBP in the hippocampus, as shown in FIG. 10, when compared with the negative control. In addition, it was confirmed that the experimental groups treated with 20 mg/kg, 40 mg/kg, and 80 mg/kg of mumefural had similar effects to those of the normal control.

Based on the results, the mumefural normalizes oligodendrocytes in the hippocampus, fornix, medial septum, corpus callosum, and fimbria hippocampi by normalizing myelin degraded due to brain damage and normalizes expression of the protein of myelin in the hippocampus in the dementia-mimic rats, and thus it was confirmed that mumefural may be effectively and safely used in prevention, treatment, and the like of the cognitive impairment-related disease such as memory impairment, Alzheimer's disease, and dementia.

### Example 5. Confirmation of Effects of Mumefural on Normalization of Synaptic Marker

In order to investigate effects of mumefural on normalization of synaptic markers in the dementia-mimic animal model, mumefural was administered in the same manner as in Example 1, and then expression levels of PSD-95 and synaptophysin-1 proteins in the hippocampus were analyzed.

Specifically, the hippocampus was excised from each of the dementia-mimic rats after administration of mumefural was completed, and the proteins were extracted therefrom. Expression levels of the PSD-95 and synaptophysin-1 proteins were analyzed by western blotting. β-Actin was used as a loading control.

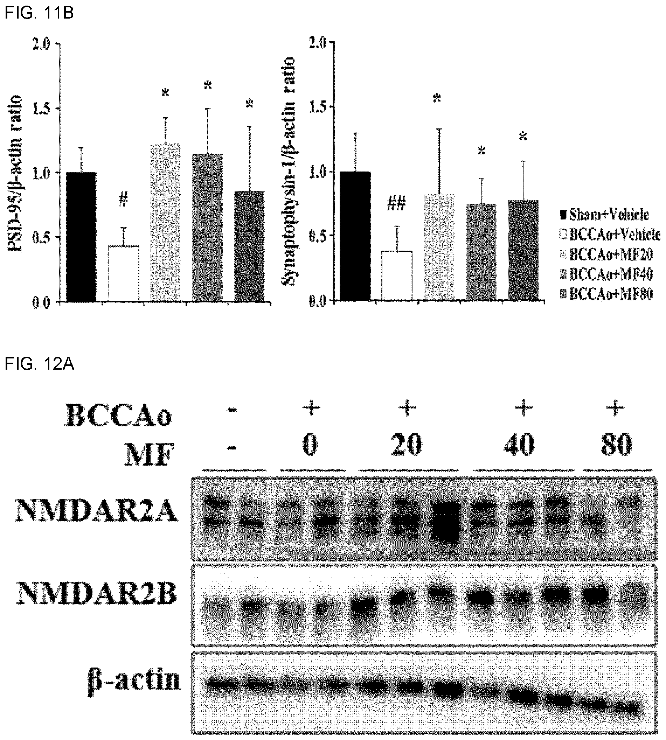

As a result, as shown in FIGS. 11A and 11B, it was confirmed that the experimental groups treated with mumefural had increased expression levels of PSD-95 and synaptophysin-1 proteins when compared with the negative control. Also, it was confirmed that the experimental groups treated with 20 mg/kg, 40 mg/kg, and 80 mg/kg of mumefural had similar effects to those of the normal control.

Based on the results, mumefural normalized synaptic markers that had been reduced by brain damage in the dementia-mimic rats, and thus it was confirmed that mumefural may be effectively and safely used in prevention, treatment, and the like of the cognitive impairment-related disease such as memory impairment, Alzheimer's disease, and dementia.

### Example 6. Confirmation of Effects of Mumefural on Normalization of Damaged Hippocampus

In order to investigate effects of mumefural on normalization of damaged hippocampus in the dementia-mimic animal model, mumefural was administered in the same manner as in Example 1, and then amounts of post-synaptic receptors in the hippocampus were analyzed by examining expression levels of *N*-methyl-D-aspartate receptor 2A (NMDAR2A) and *N*-methyl-D-aspartate receptor 2B (NMDAR2B) proteins.

Specifically, the hippocampus was excised from each of the dementia-mimic rats after administration of mumefural was completed, and the proteins were extracted therefrom. Expression levels of the NMDAR2A and NMDAR2B proteins were analyzed by western blotting. β-Actin was used as a loading control.

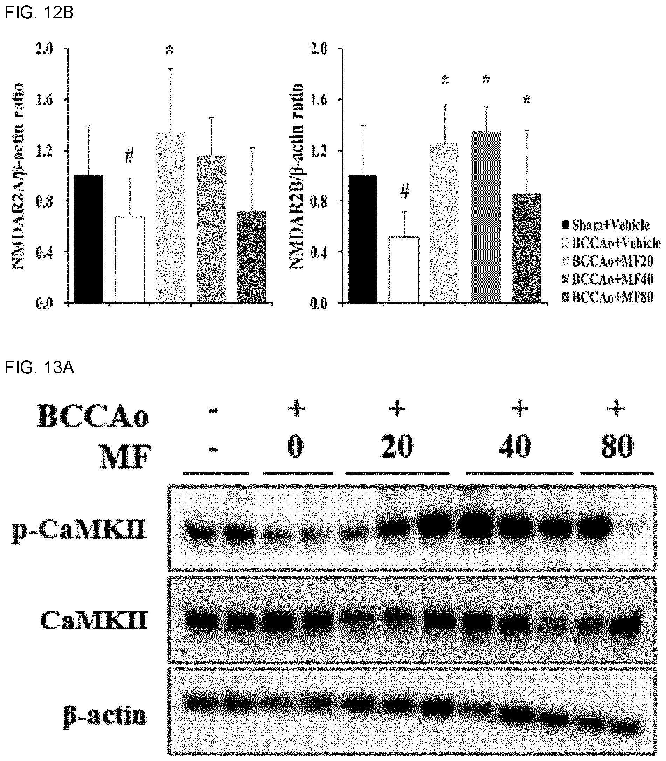

As a result, as shown in FIGS. 12A and 12B, it was confirmed that the experimental groups treated with mumefural had significantly increased expression levels of NMDAR2A and NMDAR2B protein when compared with the negative control. Also, it was confirmed that the experimental groups treated with 20 mg/kg, 40 mg/kg, and 80 mg/kg of mumefural had similar effects to those of the normal control.

Based on the results, mumefural normalized damaged hippocampus by normalizing the amounts of post-synaptic receptors in the hippocampus that had decreased due to brain damage in the dementia-mimic rats, and thus it was confirmed that mumefural may be effectively and safely used in prevention, treatment, and the like of the cognitive impairment-related disease such as memory impairment, Alzheimer's disease, and dementia.

### Example 7. Confirmation of Effects of Mumefural on Normalization of Damaged Hippocampus

In order to investigate effects of mumefural on normalization of damaged hippocampus in the dementia-mimic animal model, mumefural was administered in the same manner as in Example 1, and then expression levels of neurotransmitter secretion substances in the hippocampus were analyzed.

Specifically, the hippocampus was excised from each of the dementia-mimic rats after administration of mumefural was completed, and proteins were extracted therefrom. Expression levels of phospho-Ca²⁺/calmodulin-dependent protein kinase II (phospho-CAMKII) and Ca²⁺/calmodulin-dependent protein kinase II (CAMKII) proteins were analyzed by western blotting. β-Actin was used as a loading control.

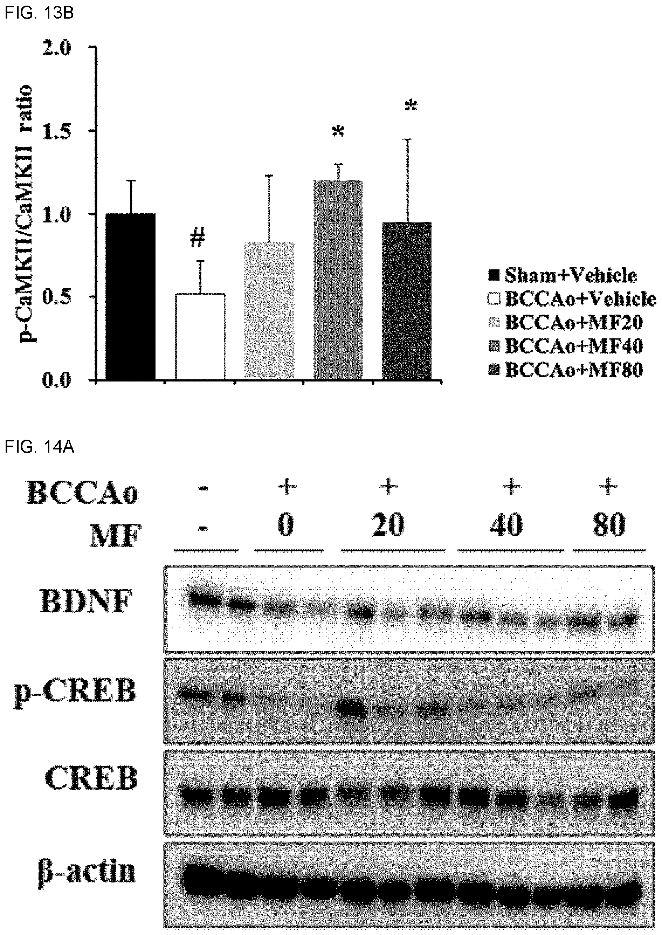

As a result, as shown in FIGS. 13A and 13B, it was confirmed that the experimental groups treated with mumefural had significantly increased expression levels of the phospho-CAMKII protein when compared with the negative control. Also, it was confirmed that the experimental groups treated with 20 mg/kg, 40 mg/kg, and 80 mg/kg of mumefural had similar effects to those of the normal control.

Based on the results, mumefural normalized damaged hippocampus by increasing the amounts of the neurotransmitter secretion substances in the hippocampus that had decreased due to brain damage in the dementia-mimic rats, and thus it was confirmed that mumefural may be effectively and safely used in prevention, treatment, and the like of the cognitive impairment-related disease such as memory impairment, Alzheimer's disease, and dementia.

### Example 8. Confirmation of Effects of Mumefural on Normalization of Damaged Hippocampus

In order to investigate effects of mumefural on normalization of damaged hippocampus in the dementia-mimic animal model, mumefural was administered in the same manner as in Example 1, and then expression levels of memory-related factors in the hippocampus were analyzed.

Specifically, the hippocampus was excised from each of the dementia-mimic rats after administration of mumefural was completed, and proteins were extracted therefrom. Expression levels of brain-derived neurotrophic factor (BDNF), phospho-cAMP response element-binding protein (phospho-CREB), and cAMP response element-binding protein (CREB) were analyzed by western blotting. β-Actin was used as a loading control.

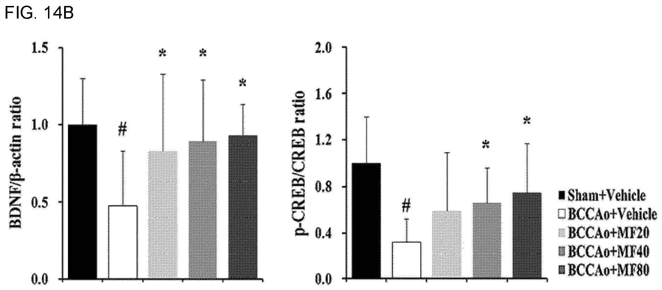

As a result, as shown in FIGS. 14A and 14b, it was confirmed that the experimental groups treated with mumefural had significantly increased expression levels of BDNF and phospho-CREB proteins when compared with the negative control. In particular, it was confirmed that the experimental groups treated with 20 mg/kg, 40 mg/kg, and 80 mg/kg of mumefural had higher expression levels than that of the normal control by about three times or more.

Based on the results, mumefural normalized damaged hippocampus by increasing the expression levels of memory-related factor in the hippocampus that had decreased due to brain damage in the dementia-mimic rats, and thus it was confirmed that mumefural may be effectively and safely used in prevention, treatment, and the like of the cognitive impairment-related disease such as memory impairment, Alzheimer's disease, and dementia.

### Example 9. Confirmation of Effects of Mumefural on Normalization of Gliosis

In order to investigate effects of mumefural on normalization of damaged hippocampus and corpus callosum of white matter in the dementia-mimic animal model, mumefural was administered in the same manner as in Example 1, and then microglia and astrocytes were detected in the hippocampus and white matter to identify gliosis. Meanwhile, microglia serving as a phagocyte of wastes of nervous tissue removes denatured neurons or foreign materials from tissue, and astrocytes serve to reconstruct damaged neurons. Thus, increases in microglia or astrocytes may be considered as occurrence of brain damage.

Specifically, the brain was excised from each of the dementia-mimic rats after administration of mumefural was completed and sectioned into slices with a thickness of 40 µm. Subsequently, in order to detect microglia in the hippocampus and white matter, an ionized calcium-binding adaptor molecule (lba-1) protein expressed in the cells was used. Also, in order to detect astrocytes, a glial fibrillary acidic protein (GFAP) expressed in the cells was used. The slices were stained by immunohistostaining using anti-lba-1 antibody or anti-GFAP antibody as a primary antibody and horse anti-mouse antibody as a secondary antibody.

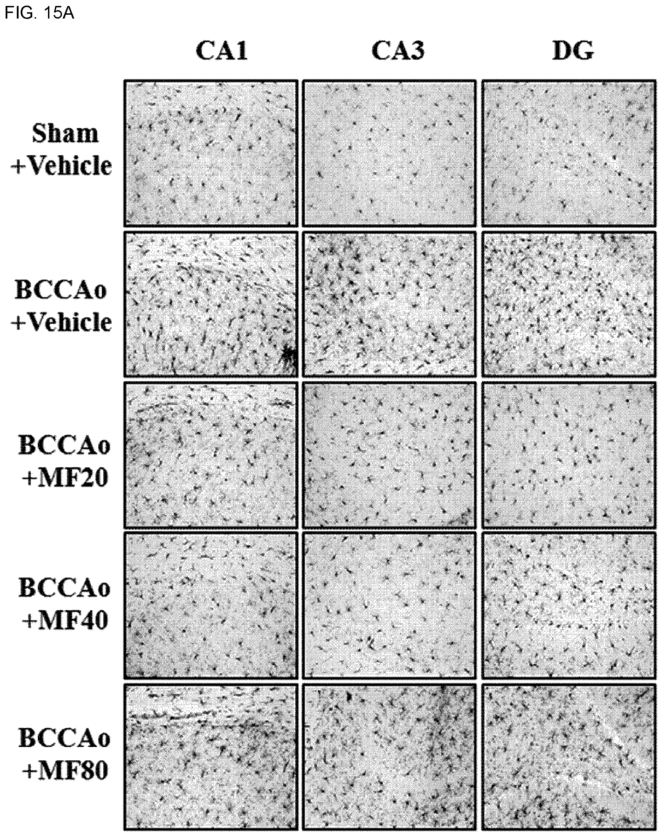

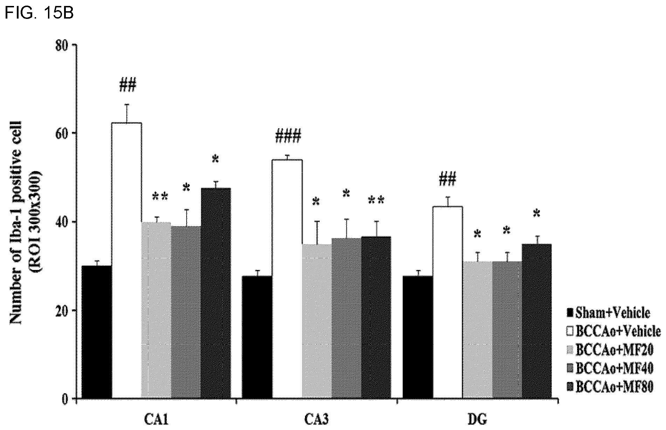

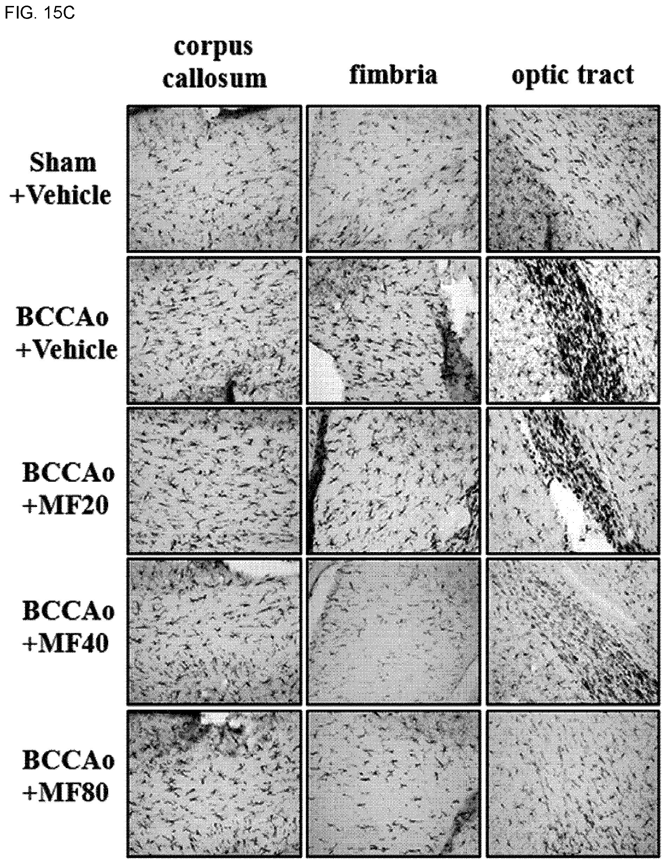

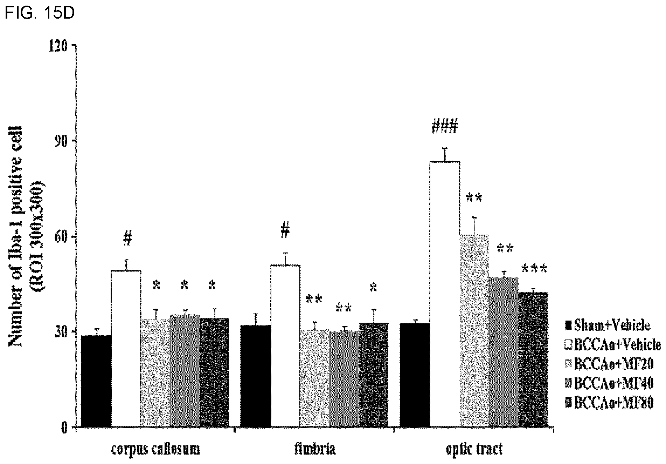

As a result, as shown in FIGS. 15A to 15D, it was confirmed that the experimental groups treated with mumefural had significantly decreased numbers of microglia in the hippocampus (FIGS. 15A and 15B) and in the white matter (FIGS. 15C and 15D) when compared with the negative control. In particular, it was confirmed that the experimental groups treated with 20 mg/kg, 40 mg/kg, and 80 mg/kg of mumefural had similar effects to those of the normal control.

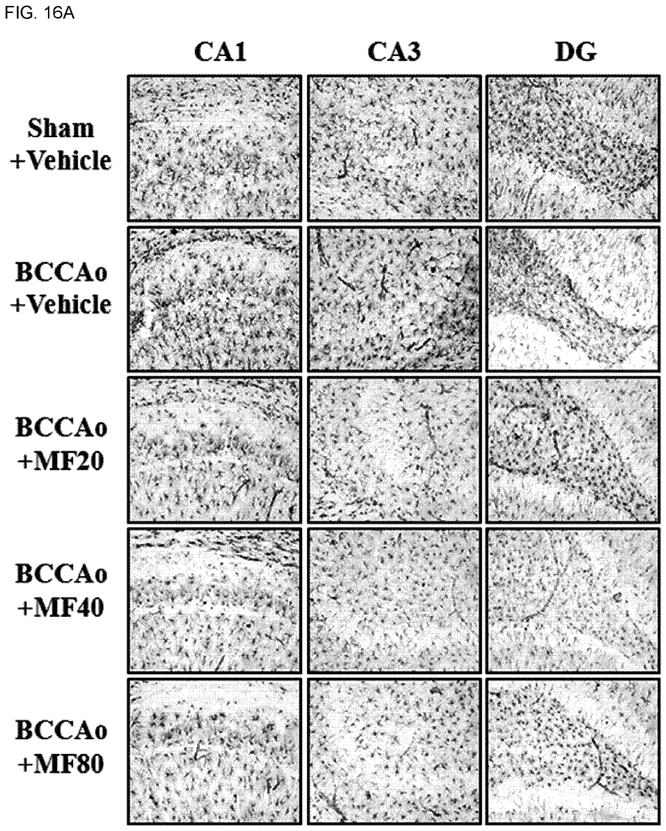

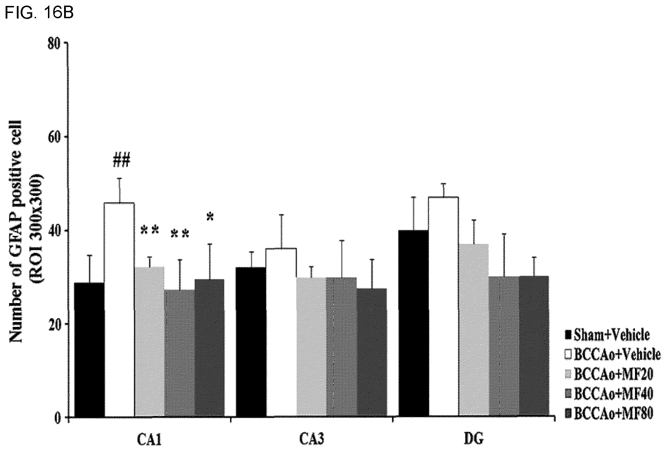

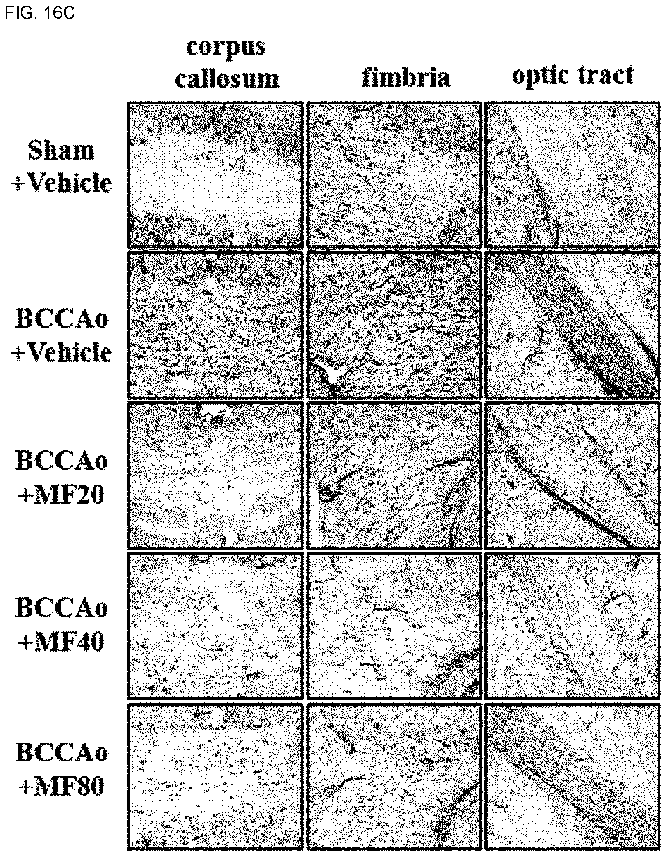

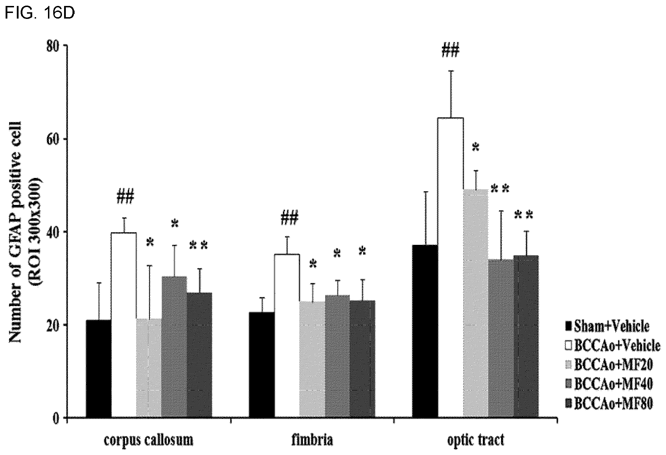

Also, as shown in FIGS. 16A to 16D, it was confirmed that experimental groups treated with mumefural had significantly decreased numbers of astrocytes in the hippocampus (FIGS. 16A and 16B) and in the white matter (FIGS. 16C and 16D) when compared with the negative control. In particular, it was confirmed that the experimental groups treated with 20 mg/kg, 40 mg/kg, and 80 mg/kg of mumefural had effects similar to or superior to those of the normal control.

Based on the results, mumefural reduced damage to cerebral nerves in the brain by normalizing damaged hippocampus and white matter in the dementia-mimic rats, and thus it was confirmed that mumefural may be effectively and safely used in prevention, treatment, and the like of the cognitive impairment-related disease such as memory impairment, Alzheimer's disease, and dementia.

### Example 10. Confirmation of Effects of Mumefural on Reduction of Neurological Inflammation

In order to investigate effects of mumefural on reduction of neurological inflammation in the dementia-mimic animal model, mumefural was administered in the same manner as in Example 1, and then expression levels of P2X purinoceptor 7 (P2X7R), toll-like receptor 4 (TLR4), myeloid differentiation primary response 88 (MyD88), nucleotide-binding oligomerization domain-like receptor protein 3 (NLRP3), caspase1, interleukin (IL)-1β, IL-18, phospho-signal transducer and activator of transcription 3 (phospho-STAT3), and NF-κB(p65, p50) proteins were measured in the hippocampus. Meanwhile, these proteins provoke inflammation of nerve cells, causing damage to the brain and resulting in diseases such as dementia.

Specifically, the hippocampus was excised from each of the dementia-mimic rats after administration of mumefural was completed, and proteins were extracted therefrom. Expression levels of the proteins were analyzed by western blotting. β-Actin and LaminB1 were used as loading controls.

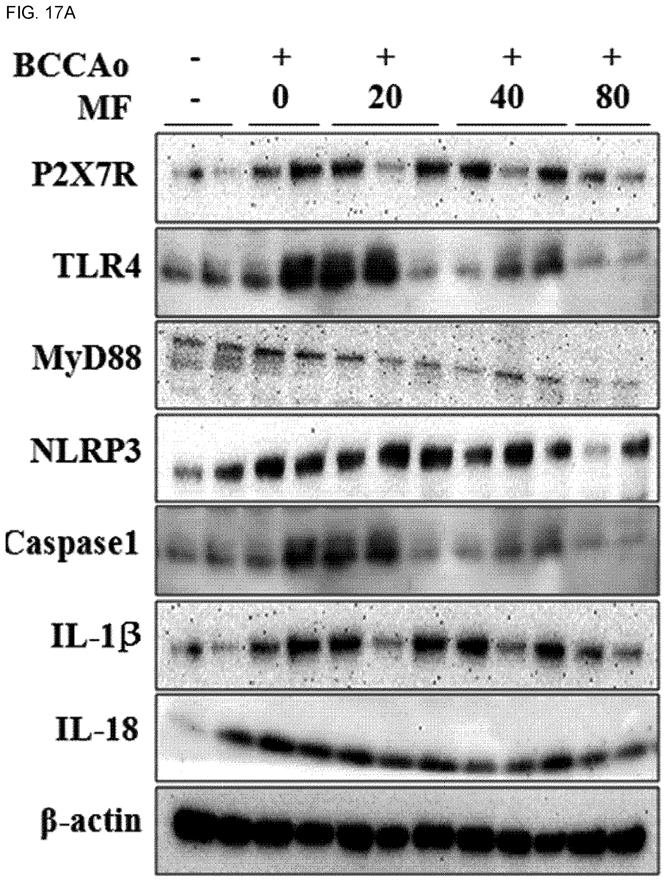

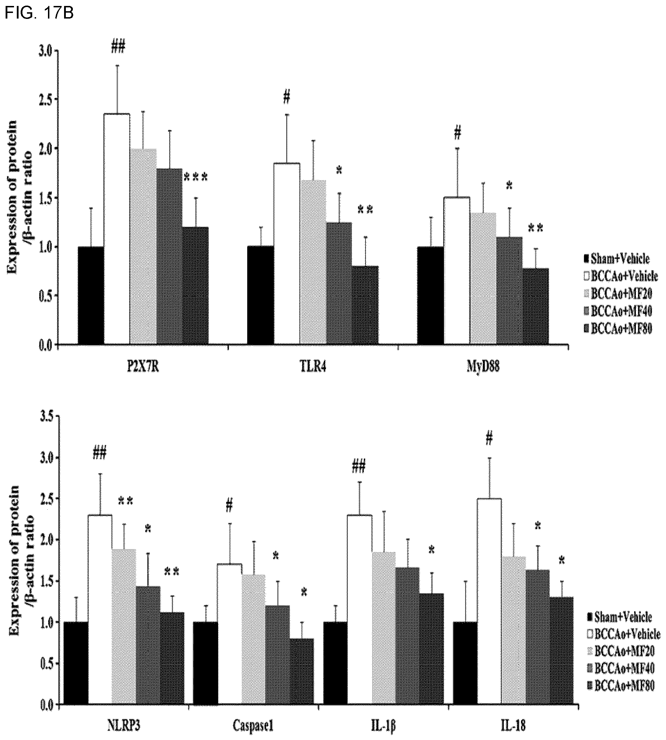

As a result, as shown in FIGS. 17A to 19B, it was confirmed that the experimental groups treated with mumefural had decreased expression levels of P2X7R, TLR4, MyD88, NLRP3, caspase1, IL-1β, IL-18, phospho-STAT3, and NP-κB(p65, p50) proteins when compared to the negative control. In particular, it was confirmed that the experimental groups treated with 20 mg/kg, 40 mg/kg, and 80 mg/kg of mumefural had effects similar to or superior to those of the normal control.

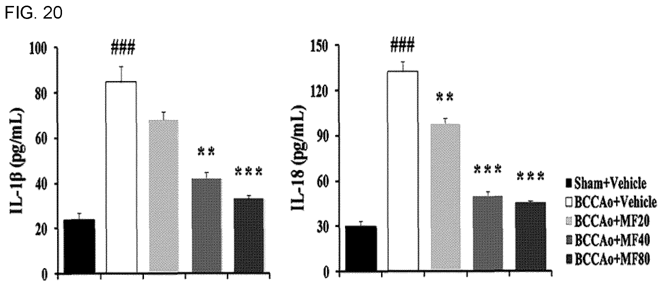

Also, as shown in FIG. 20, based on enzyme immunoassay results of IL-1β and IL-18, which are inflammatory cytokines, for identification of the levels of inflammatory cytokines in hippocampus tissue, it was confirmed that the experimental groups treated with mumefural had significantly decreased expression levels of IL-1β and IL-18 when compared with the negative control.

Based on the results, mumefural reduced neurological inflammation caused by brain damage in the dementia-mimic rats, and thus it was confirmed that mumefural may be effectively and safely used in prevention, treatment, and the like of the cognitive impairment-related disease such as memory impairment, Alzheimer's disease, and dementia.

The above description of the present invention is provided for the purpose of illustration, and it would be understood by those skilled in the art that various changes and modifications may be made without changing the technical conception and essential features of the present invention. Thus, it is clear that the above-described embodiments are illustrative in all aspects and do not limit the present invention. The various embodiments disclosed herein are not intended to be limiting, with the true scope and spirit being indicated by the following claims. The present invention is to be limited only by the terms of the appended claims, along with the full scope of equivalents to which such claims are entitled.

## Claims

1. A pharmaceutical composition for preventing or treating a cognitive impairment-related disease, the pharmaceutical composition comprising mumefural or a pharmaceutically acceptable salt thereof as an active ingredient.

2. The pharmaceutical composition of claim 1, wherein the mumefural is represented by Formula 1 below:

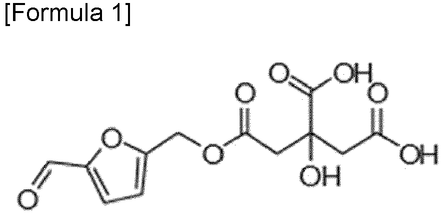

3. The pharmaceutical composition of claim 1, wherein the cognitive impairment-related disease comprises at least one selected from the group consisting of dementia, Alzheimer's disease, and memory impairment.

4. The pharmaceutical composition of claim 3, wherein the dementia is vascular dementia or Alzheimer's dementia.

5. The pharmaceutical composition of claim 1, wherein the composition comprises 0.01 wt% to 80 wt% of the compound.

6. The pharmaceutical composition of claim 1, wherein the composition further comprises a pharmaceutically acceptable carrier.

7. A method of treating a cognitive impairment-related disease, the method comprising administering the composition of any one of claims 1 to 6 to an individual suspected of having a cognitive impairment-related disease.

8. A health functional food composition for preventing or alleviating a cognitive impairment-related disease, the health functional food comprising mumefural or a physiologically acceptable salt thereof as an active ingredient.

9. A health functional food composition for enhancing learning ability, cognitive function, or memory, the health functional food comprising mumefural or a physiologically acceptable salt thereof as an active ingredient.

10. A feed composition for preventing or alleviating a cognitive impairment-related disease, the feed composition comprising mumefural or a physiologically acceptable salt thereof as an active ingredient.
